# EUROPEAN PATENT APPLICATION

(11) **EP 0 729 023 A1**
(43) Date of publication of application: **28.08.1996**
(21) Application number: 95120431.2
(22) Date of filing: 22.12.1995
(51) Int. Cl.: G01N 21/31, A61B 5/083

(54) **Optical analyser**

(30) Priority: 27.02.1995 SE 9500712
(71) Applicant: Siemens Elema AB, S-171 95 Solna 1 (SE)
(72) Inventor: Ollson, Sven-Gunnar, S-232 00 Arlöv (SE); Rydgren, Göran, S-230 44 Bunkeflostrand (SE); Brauer, Stefan, S-224 73 Lund (SE); Linge, Anders, S-244 36 Kävlinge (SE)

(57) **Abstract**

The present invention relates to an optical analyser (2) capable of performing simultaneous measurement in a first test chamber (12) and a second test chamber (22). The analyser (2) has a first radiation path (4) and a second radiation path (18), the first test chamber (12) being placed in the first radiation path (4) and the second test chamber (22) being placed in the second radiation path (18). Radiation in the first radiation path (4) is generated by a first source of radiation (6), and radiation in the second radiation path (18) is generated by a second source of radiation (20). A filter device (18), containing a number of optical filters (10C, G), filters out specific wavelengths which are to pass through the respective test chamber (12, 22). The filter device (8) rotates so the optical filters (10C, G) are alternately placed in the first radiation path (4) and the second radiation path (18) respectively. Radiation passing the first test chamber (12) is registered by a first radiation detector (14), and a detector signal is sent to an analysis unit (16) which identifies or determines the concentration of the specific substance in the first test chamber (12). In the corresponding manner, radiation passing the second test chamber (22) is detected by a second radiation detector (24), which also send the detector signals to the analysis unit (16), so the corresponding analysis of the specific substance in the second test chamber (22) is performed.

## Description

The present invention relates to an optical analyser for identifying a specific substance in at least two mixtures of substances and/or for measuring the concentration of the specific substance, comprising a first radiation path in which a first test chamber, containing a first mixture of substances, is arranged and in which a filter device, comprising at least two optical filters, each passing through a specific radiation wavelength, the specific substances absorbing radiation at at least one of the wavelengths, is arranged so the optical filters are alternately placed in the first radiation path, the specific substance being identified and/or the concentration of the specific substance being determined from the specific substance's absorption of radiation at the different wavelengths.

Spectrophotometry is a common optical analysis method. It can be used for identifying all substances which absorb radiation at at least some unique wavelength (which normally lies in the light spectrum between infrared and ultra violet). The method can also be used for measuring the concentration of the substances. The absorption of light by these substances normally occurs at several specific wavelengths, so the absorption spectrum of each substance constitutes a unique "fingerprint". In determinations of the absorption, radiation of a narrow wavelength range is sent through a test chamber or test cell containing a sample, and the passing radiation is measured. A reference value, which can be obtained by sending the same radiation through another test chamber, containing a substance which does not absorb the radiation, is needed in order to quantify the absorption on the basis of the measured radiation. Alternately, radiation of a wavelength, at which the sought substance does not absorb any radiation, can be sent through the test chamber, and the passing radiation can be measured. In those instances where a reference sample is used, the sample containers can be shifted so they alternately are in the radiation path, or two radiation paths can be used so as to obtain a reference value and measurement signal at the same time. Devising this equipment to enable it to measure two test mixtures simultaneously would result in needlessly complex and expensive equipment designs. Especially when measurements in real time are desired.

WO-A-81/0062 describes an optical analyser which employs radiation at two wavelengths in order to obtain a reference signal and a measurement signal. Beams of radiation are emitted from a radiation source in two directions, and each of the beams passes an interference filter which filters out all radiation except radiation with a specific wavelength. With the aid of a system of mirrors, the two beams obtained are directed to pass through a test chamber and then strike a radiation detector. A mechanical chopper is arranged in the path of both beams to keep signals from the two filtered radiation beams from striking the detector at the same time. The mechanical chopper consists of a rotatable disc with openings, which only allow one wavelength at a time to enter the test chamber and strike the radiation detector. The concentration of the substance can subsequently be determined by an analysis unit from the ratio between the reference signal and the measurement signal. This device is also unable to simultaneously measure two samples.

Another optical analyser is described in EP-A-0 385 256. This analyser is intended for real time measurement of the carbon dioxide content in air expired by a patient. Here, the patient breathes directly through the test tube, so all expired air passes through the tube. Light from a radiation source passes through the tube and a filter device. The filter device is devised as a rotating disk on which two optical filters are arranged, each filter passing a specific wavelength from the radiation passing the test chamber. A radiation detector then measures the radiation at the two wavelengths, and the carbon dioxide content of the expired air is determined from the ratio between radiation at the respective wavelengths.

The ability to measure the concentration of different gases in real time is desirable in ventilator and anaesthetic systems, especially in anaesthesia systems where a plurality of gases is normally employed. For instance, the uptake of anaesthetic gas in the patient could be determined if the concentration of anaesthetic gas supplied to the patient and the concentration of anaesthetic gas leaving the patient are determined. As an additional safety measure, reliable identification of the anaesthetic gas would also be useful in ensuring that the concentration of the anaesthetic gas supplied to the patient is correct and not harmful to the patient.

As the cited document EP-A-0 385 256 shows, measuring the carbon dioxide concentration in the patient's expiratory air at a Y piece connected to the patient is known. The design of this Y piece is critical. In particular, its volume should not be too large, since dead space must not become too large. The risk of this increases if excessively comprehensive measurement equipment is arranged at the Y piece.

Other known measurement systems instead extract a small gas sample from the Y piece in order to perform measurements on the sampled gas. Samples are then extracted both when gas is supplied to and removed from the patient. One common disadvantage for all these analysers is that the equipment must be located very close to the patient to yield good measurement values, especially to avoid time delays in the measurement result. This creates a problem for both the patient and staff. Another disadvantage is that a sample extracted from the patient's Y piece disrupts the flow of gas to the patient. In order to minimise these disruptions, the extracted sample is sometimes returned to the Y piece. But then extraction of an erroneous gas mixture as a sample is a risk instead. This because the gas flow changes direction, and a sample previously extracted could therefore get mixed with a new sample from the Y piece.

One object of the invention is to achieve an optical analyser capable of performing measurements of at least two mixtures of substances at the same time.

Another object of the invention is to achieve an optical analyser capable of performing a plurality of real time measurements on at least two mixtures at the same time.

Another objective of the invention is to achieve an optical analyser for ventilator/anaesthetic systems in which the above-mentioned disadvantages and problems of these systems are avoided.

One such optical analyser is achieved in accordance with the invention in that the optical analyser further comprises a second radiation path in which a second test chamber, containing a second mixture of substances, is arranged, and the filter device is arranged so the optical filters are alternately placeable also in the second radiation path in order to identify the specific substance and/or determine the concentration of the specific substance in the second test chamber.

With this design for the optical analyser, two completely separate samples can be analysed at the same time for one and the same specific substance. The same filter device is used for both radiation paths, thereby greatly reducing the cost of the analyser, since the filter device represents the highest cost in such equipment. An additional advantage is that the exact same wavelengths pass through both test chambers when the respective filter is placed in the respective radiation path.

A third and forth and further radiation path and test chamber can be arranged next to the first and second. All of these utilising the same filter device.

It is advantageous if the first radiation path comprises a first source of radiation for generating radiation and a first radiation detector for detecting radiation passing the first test chamber and the filter device, then generating corresponding detector signals, and if an analysis unit is connected to the first radiation detector for identifying and/or determining the concentration of the specific substance.

The second radiation path can be devised in different ways. One first design is achieved in accordance with the invention in that the second radiation path comprises a second source of radiation for generating radiation and a second radiation detector for detecting radiation passing the second test chamber and the filter device, then generating corresponding detector signals, and the second radiation detector is connected to the analysis unit. In other words a doubling of the components in the first radiation path. Radiation sources and radiation detectors are normally inexpensive, making for a simple and economical analyser design.

Alternately, the second radiation path can be devised by having a first system of mirrors diverting radiation from the first radiation path to form the second radiation path and a second system of mirrors diverting radiation passing the second test chamber and the filter device to the first radiation detector. With such a design, it will be necessary to alternately measure radiation through the first test chamber and the second chamber respectively to avoid interference between the two samples.

It is, of course, also possible to devise combinations of these two designs. For example, the second radiation path could have a mirror system for diverting radiation from the first radiation path and further have a separate radiation detector for the radiation which then passes through the second test chamber. Conversely, radiation in the second radiation path could be generated by a separate source of radiation, and a system of mirrors can divert the radiation passing through the second test chamber to the first radiation detector.

One embodiment of the analyser is achieved in accordance with the invention in that the first test chamber is connected to a first line in which the first mixture of substances flows, and the second test chamber is connected to a second line in which the second mixture of substances flows.

This design conveys the advantage that each sample to be analysed is replaced with a fresh sample the whole time. This makes possible continuous real time analysis of flowing substances.

Here, it is advantageous if the first test chamber is an integral part of the first line and the second test chamber is an integral part of the second line, identification and/or determination of the concentration of the specific substance then being performed on the entire flow or the passing flow respectively.

This is especially advantageous in the identification or determination of the concentration of substances with low absorption at the measured wavelengths or is only present in a very low concentration, therefore making it necessary for the largest possible part of the test mixture to be irradiated in the radiation path. This is especially the case when the first mixture of substances and the second mixture of substances consist of gas mixtures. This design for the optical analyser conveys a particular advantage when the first line consists of an inspiratory tube for a ventilator/anaesthetic system and the second line consists of the expiratory tube for a ventilator/anaesthetic system.

All measurements of gases to and from the patient can accordingly be performed at a distance from the patient. This will achieve the second object of the invention, and it will no longer be necessary to set up equipment near the patient and impede the physician's access to the patient. The connection of a suitable means for carrying gas to and from the patient, such as a tracheal tube, is all that is necessary. Since measurements can be performed both on gas delivered to the patient and on gas carried away from the patient, uptake of the specific gas in the patient can be easily determined by the analyser.

An improvement of the optical analyser is achieved in accordance with the invention in that the filter device is devised with at least a third optical filter, which is alternately, with the other optical filters, placed in the first radiation path and the second radiation path, thereby making it possible to identify and/or determine the concentration of at least one additional specific substance in the test chambers. In principle, an optional number of optical filters can be arranged in the filter device so they are alternately placed in the respective radiation path. The only real limitation here is the time the test chambers must be irradiated at the respective radiation wavelength to achieve reliable determination of the specific substance. The uptake of different gases in the patient can then be easily determined.

One advantageous way to make the optical filters placeable in the radiation paths is achieved in accordance with the invention in that the filter device comprises at least one disk, which is rotatably arranged between the first test chamber and the second test chamber, and a drive unit, connected to the analysis unit, for rotating the disk so the optical filters are placed, one by one, in the respective radiation path for predetermined periods of time.

When several discs containing optical filters are used, it is preferable to have to two opposite arranged openings on each disc. When the filters on one disc is not in use, the disc is positioned so the openings are placed in the radiation paths, thereby not disturbing measurements with optical filters on the other disc.

It is also advantageous if the filter device is placed before the test chambers in the respective radiation path, whereupon only the specific wavelengths passed by the optical filters are passing through the test chambers. Any interference, caused by the excitation of other substances in the mixture and emission of radiation in the measured wavelength range, is accordingly minimised.

Two embodiments of the optical analyser according to the invention will now be described in greater detail, referring to the figures in which
FIG. 1 shows a first embodiment of the optical analyser;
FIG. 2 shows the first embodiment from another angle;
FIG. 3 shows one use of the optical analyser in conjunction with a ventilator/anaesthetic system;
FIG. 4 shows a second embodiment of the optical analyser;
FIG. 5 shows a third embodiment of the optical analyser; and
FIG. 6 shows a fourth embodiment of the optical analyser.

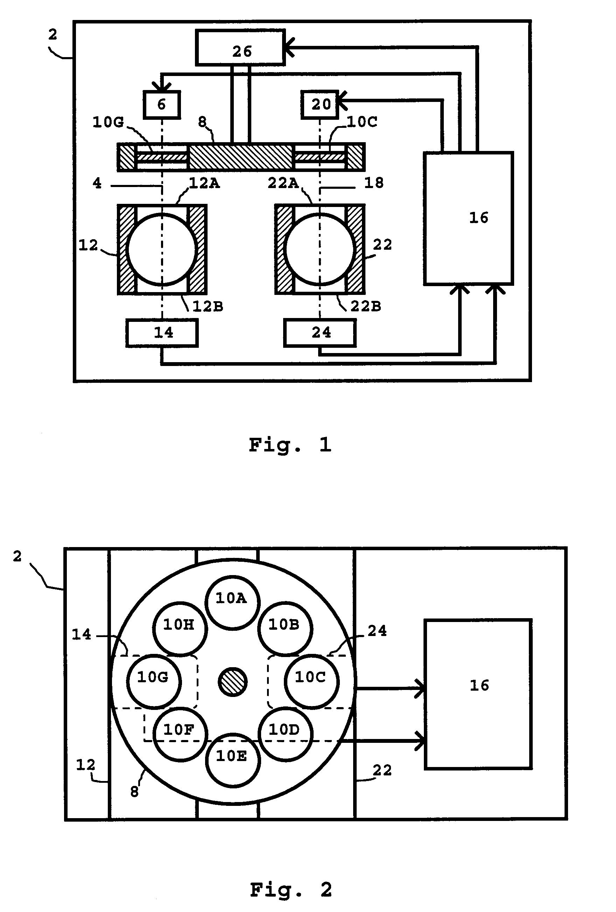

The following will refer both to FIG. 1 and FIG. 2. An optical analyser 2 comprises a first radiation path 4 whose radiation is generated by a first source of radiation 6. The radiation passes a filter device 8 in which eight filters 10A-H are arranged. The filter device 8 rotates so the filters 10A-H are alternately placed in the first radiation path 4, each filter then filtering out a specific wavelength. It is also possible for one of the optical filters 10A-H to completely block radiation from the first source of radiation 6. Such blocking of radiation facilitates zeroing of detectors having a drift.

The specific radiation passing the respective optical filter 10A-H then passes through a first test chamber 12. The first test chamber has a first sapphire window 12A and a second sapphire window 12B, which pass radiation passing the filter device 8. The test chamber has a first mixture of substances containing the specific substance whose identity and/or concentration is sought. After passing the first test chamber 12, the radiation strikes a first radiation detector 14, thereupon generating a detector signal which is sent to an analysis unit 16. The analysis unit 16 also serves as a control unit and source of power for the components in the analyser 2.

A second radiation path 18 is arranged parallel to the first radiation path 4. A second source of radiation 20 generates radiation for the second radiation path 18. Radiation from the second source of radiation 20 passes the filter device 8 and a second test chamber 22. Like the first test chamber 12, the second test chamber 22 is devised with a first sapphire window 22A and a second sapphire window 22B to pass radiation to a second mixture of substances containing the specific substance. The radiation passing the second test chamber 22 is detected by a second radiation detector 24 which generates the corresponding detector signals and send them to the analysis unit 16.

In this manner, virtually simultaneous identification and/or determination of the concentration of at least one specific substance in the respective test chamber 12, 22 can be performed. In principle, the optical filters 10A-H are selected so there is one optical filter for each specific substances whose identity and/or concentration are sought, which optical filter passes a radiation wavelength at which the specific substance does not absorb radiation and at least one optical filter which passes radiation absorbed by the specific substance. Detection of absorption at a plurality of wavelengths may be necessary for certain substances. This is the case for e.g. anaesthetic gases whose absorption spectra are very similar to a large degree.

The filter device is devised as a disk so measurement in the optical analyser 2 can be performed in the most efficient manner possible. With the aid of a drive unit 26, the filter device 8 can be rotated so the optical filters 10A-H are placed, one after another, in the respective radiation path 4, 18.

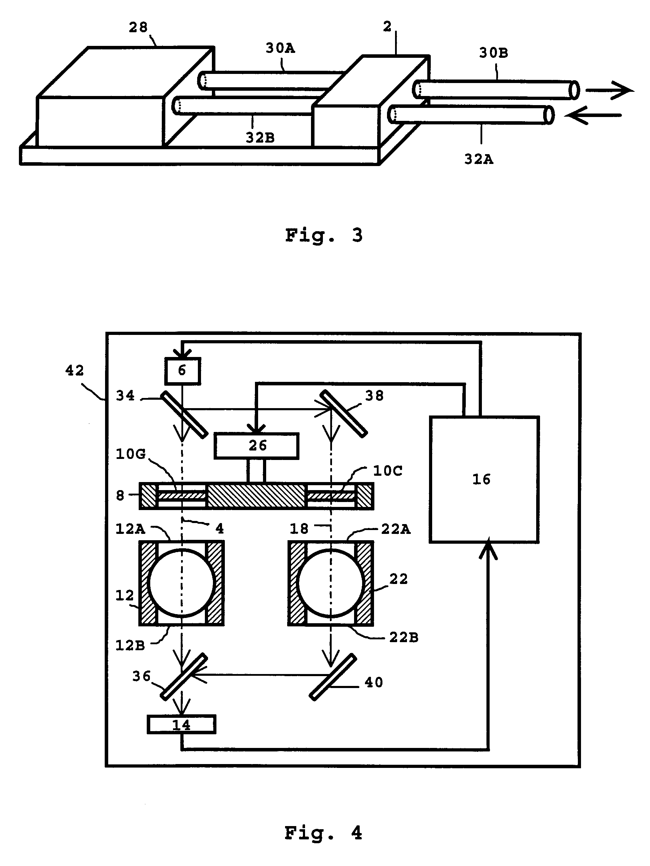

FIG. 3 shows an advantageous use of the analyser 2 with a ventilator/anaesthetic system 28. Gas is carried from the ventilator/anaesthetic system 28 to a patient via an inspiratory tube 30. The inspiratory tube 30 is divided into a first section 30A and a second section 30B, the analyser 2 being placed between these parts 30A, 30B. The concentration of the gases supplied to the patient can thereby be determined.

Gas expired by the patient is returned to the ventilator/anaesthetic system 28 via an expiratory tube 32. The expiratory tube 32 also consists of a first section 32A and a second section 32B, the analyser 2 being placed between the two parts 32A, 32B. The concentration of the gases removed from the patient is measured in the expiratory tube.

When the concentration of a specific anaesthetic gas supplied to and removed from the patient is compared, the uptake of anaesthetic gas in the patient can be established. This also applies to other gases, such as nitrous oxide and other gases, supplied to and removed from the patient. It can also be used for measuring e.g. the carbon dioxide exhaled by the patient. Measurement of carbon dioxide in closed anaesthetic systems with rebreathing of expired gas after filtration represents an additional safety check ensuring that inspired gas does not contain excessive amounts of carbon dioxide. Even in ventilator systems employing ordinary air, measurement of the carbon dioxide content of both inspired and expired air is advantageous in determining the patient's carbon dioxide output.

Placing the analyser 2 closer to the ventilator/anaesthetic system 28 makes things easier for both the doctor and the patient, since only a small part of the equipment needs to be located close to the patient. There is also the additional advantage that measurement is performed on the entire passing gas flow, so no gas samples need to be taken, and there is no disruption of any other kind in the passage of gas to and from the patient. This will also minimise dead space. If flow is also measured during determinations of concentration, the volumes of the respective gases can easily be determined.

FIG. 4 shows a second embodiment of the optical analyser according to the invention. Components here which are identical to components in the first embodiment have been assigned the same reference numbers.

The optical analyser 42 thus comprises a first radiation path 4 and a second radiation path 18. As in the first embodiment, the first radiation path 4 is generated by a first source of radiation 6. Radiation from the first source of radiation 6 then passes a first semi-transparent mirror 34, and the beam is split into two parts. The first part of the beam, which consists of the first radiation path 4, then passes a filter device 8 and a first test chamber 12. A first sapphire window 12A and a second sapphire window 12B are arranged in the first test chamber 12 to pass radiation through the mixture of substances in the first test chamber 12. The radiation which passes the first test chamber 12 then passes through a second semi-transparent mirror 36 and strikes a first radiation detector 14, which is connected to an analysis unit 16.

As in the first embodiment, the filter device 8 is driven by a drive unit 26 controlled by the analysis unit 16. The analysis unit 16 also serves as a source of power for the first source of radiation 6.

The radiation reflected by the first semi-transparent mirror 34 strikes a third mirror 38 to form the second radiation path 18. The radiation reflected by the third mirror 38 passes the filter device 8 and a second test chamber 22 in which a first sapphire window 22A and a second sapphire window 22B are arranged. The radiation passing the second test chamber 22 strikes a fourth mirror 40, is reflected towards the second semi-transparent mirror 36 and then strikes the radiation detector 14. To prevent radiation passing the first test chamber 12 from striking the radiation detector 14 at the same time as radiation passing the second test chamber 22, the optical filters 10A-G should be mounted so only one radiation path 4, 18 at a time passes radiation to the first radiation detector 14.

The latter problem would be easily solved if a second radiation detector were placed in the second radiation path 18 and the mirrors 36 and 40 were eliminated.

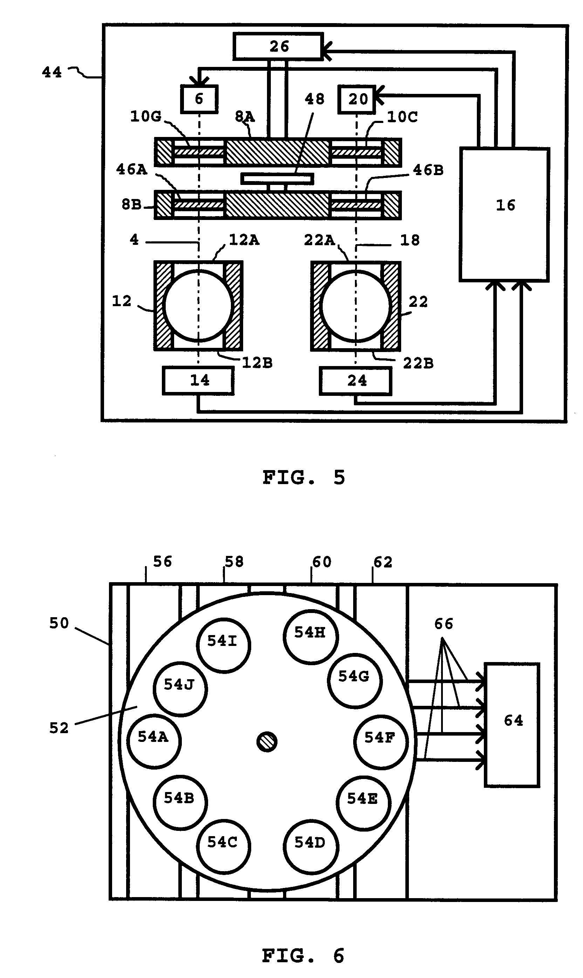

FIG. 5 shows a third embodiment of the optical analyser, designated 44. As for the first and second embodiments, elements which could be identical have retained their designation number.

Basically, the optical analyser 44 is identical with the first embodiment of FIG. 1 and FIG. 2, except for one thing. The filter device 8 comprises a first disc 8A and a second disc 8B. Both discs 8A, 8B are supplied with optical filters 10G, 10C, 46A, 46B. Hereby, a greater number of optical filters can be used, while maintaining a fairly small space requirement. The second disc 8B is separately driven by a second drive unit 48, powered and controlled by the analysis unit 16. The two discs 8A, 8B should preferably have two opposite openings without optical filters 10G, 10C, 46A, 46B so that one optical filter 10G, 10C, 46A, 46B at a time can be placed in the radiation paths 4, 18.

A fourth embodiment is shown in FIG. 6. The optical analyser 50 comprises a filter device 52 on which ten optical filters 54A-54J are arranged. A first sample is introduced to the analyser 50 via a first sample line 56, a second sample is introduced to the analyser 50 via a second sample line 58, a third sample is introduced to the analyser 50 via a third sample line 60 and a fourth sample is introduced to the analyser 50 via a fourth sample line 62.

All four samples can be analysed simultaneously in real time by using four or six radiation paths and radiation detectors (not shown). These can be identical to those in the preceding embodiments. As the filter device 52 is positioned, the first sample line 56 is analysed with the first filter 54A, the second sample line is analysed with the third filter 54C and/or the ninth filter 54I, the third sample line is analysed with the fourth filter 54D and/or the eighth filter 54H and the fourth sample line is analysed with the sixth filter 54F. Analysis is made in the analysing unit 64. Signals from the detectors are provided via signal lines 66.

Combinations of the four described embodiments of the optical analyser can be realized without difficulty. For instance, the second embodiment according to FIG. 4 or the fourth embodiment according to FIG. 6 could be combined with the third embodiment by adding one or several discs. Any of the first to third embodiments could be combined with the fourth by adding a third or fourth sample line, etc.

## Claims

1. An optical analyser (2; 42; 44; 50) for identifying a specific substance in at least two mixtures of substances and/or for measuring the concentration of the specific substance, comprising a first radiation path (4) in which a first test chamber (12), containing a first mixture of substances, is arranged and in which a filter device (8), comprising at least two optical filters (10A-H; 46A, 46B; 54A-G), each passing a specific radiation wavelength, the specific substances absorbing radiation at at least one of the wavelengths, is arranged so the optical filters (10A-H; 46A, 46B; 54A-G) are alternately placed in the first radiation path (4), the specific substance being identified and/or the concentration of the specific substance determined from the specific substance's absorption of radiation at the different wavelengths, **characterized in** that the analyser (2; 42; 50) further comprises at least a second radiation path (18) in which a second test chamber (22), containing a second mixture of substances, is arranged, and the filter device (8) is arranged so the optical filters (10A-H; 46A, 46B; 54A-G) are alternately placeable also in the second radiation path (18) in order to identify the specific substance and/or determine the concentration of the specific substance in the second test chamber (22).

2. An optical analyser according to claim 1, **characterized in** that the first radiation path (4) comprises a first source of radiation (6) for generating radiation and a first radiation detector (14) for detecting radiation passing the first test chamber (12) and the filter device (8), there generating corresponding detector signals, and an analysis unit (16) is connected to the first radiation detector (14) to identify and/or determine the concentration of the specific substance.

3. An optical analyser according to claim 2, **characterized in** that the second radiation path (18) comprises a second source of radiation (20) for generating radiation and a second radiation detector (24) for detecting radiation passing the second test chamber (22) and the filter device (8), there generating corresponding detector signals, and the second radiation detector (24) is connected to the analysis unit (16).

4. An optical analyser according to claim 2, **characterized in** that a first mirror system (34, 38) diverts radiation from the first source of radiation (6) to form the second radiation path (18) and a second mirror system (36, 40) diverts radiation passing the second test chamber (22) and filter device (8) to the first radiation detector (12).

5. An optical analyser according to any of the above claims, **characterized in** that the first test chamber is connected to a first line in which the first mixture of substances flows, and the second test chamber is connected to a second line in which the second mixture of substances flows.

6. An optical analyser according to claim 5, **characterized in** that the first test chamber is an integral part of the first line, and the second test chamber is an integral part of the second line, identification and/or determination of the concentration of the specific substance being performed on all of each passing flow.

7. An optical analyser according to claim 5 or 6, **characterized in** that the first mixture of substances and the second mixture of substances are mixtures of gases.

8. An optical analyser according to claim 7, **characterized in** that the first line consists of an inspiratory tube (30A, 30B) for a ventilator/anaesthetic system (28), and the second line consists of an expiratory tube (32A, 32B) for the ventilator/anaesthetic system (28).

9. An optical analyser of any of the above claims, **characterized in** that the filter device (8) is devised with at least a third optical filter (10A-H; 46A, 46B; 54A-G) which, with the other optical filters (10A-H; 46A, 46B; 54A-G), is alternately placed in the first radiation path (4) and the second radiation path (18), whereby at least one additional specific substances in the test chambers (12, 22) can be identified and/or its concentration determined.

10. An optical analyser according to any of the above claims, **characterized in** that the filter device (8) consists of at least one disk (8A, 8B), which is rotatably arranged between the first test chamber (12) and the second test chamber (22), and at least one drive unit (26, 48), connected to the analysis unit (16), for rotating the disk (8A, 8B) so the optical filters (10-H, 46A, 46B; 54A-G) are placed, one by one, in the respective radiation path (4, 18) for predetermined periods of time.

11. An optical analyser according to any of the above claims, **characterized in** that the filter device (8) is placed before the test chambers (12, 22) in the respective radiation path (4, 18), only the specific wavelengths passed by the optical filters (10A-H, 46A, 46B; 54A-G) then passing through the test chambers (12, 22).
